## Europäisches Patentamt

## European Patent Office

## Office européen des brevets

(19)

(11) Veröffentlichungsnummer: **0 184 555**
**A2**

(12) # EUROPÄISCHE PATENTANMELDUNG

(21) Anmeldenummer: **85810569.5**

(22) Anmeldetag: **02.12.85**

(51) Int. Cl.⁴: **C 07 C 113/04**

(30) Priorität: **06.12.84 CH 5825/84**

(43) Veröffentlichungstag der Anmeldung:
**11.06.86 Patentblatt 86/24**

(84) Benannte Vertragsstaaten:
**BE CH DE FR GB IT LI**

(71) Anmelder: **CIBA-GEIGY AG**
**Klybeckstrasse 141**
**CH-4002 Basel(CH)**

(72) Erfinder: **Oxenius, Rüdiger, Dr.**
**Brunnenweg 15**
**D-7888 Rheinfelden(DE)**

(72) Erfinder: **Bürgi, Ernst**
**Gwändweg 19**
**CH-4143 Dornach(DE)**

(72) Erfinder: **Arnold, Vladimir, Dr.**
**Landskronstrasse 23**
**CH-4056 Basel(CH)**

(72) Erfinder: **Rakoczi, Ferenc, Dr.**
**Grünauring 20/125**
**CH-8064 Zürich(CH)**

(54) Verfahren zur Diazotierung von Aminen.

(57) Zur Diazotierung von in Wasser schwerlöslichen Aminen werden diese als Schmelze oder Lösung in einem organischen Lösungsmittel durch Zugabe von Wasser ausgefällt und anschliessend diazotiert. Bei dieser Arbeitsweise sind die Reaktionszeiten für die Diazotierung deutlich kürzer als nach dem üblichen Verfahren.

EP 0 184 555 A2

CIBA-GEIGY AG

Basel (Schweiz)                                          1-15179/=

Verfahren zur Diazotierung von Aminen

Gegenstand der vorliegenden Erfindung ist ein Verfahren zur Diazotierung von in Wasser schwerlöslichen primären aromatischen Aminen.

Die Diazotierung von primären aromatischen Aminen wird in grossem
Umfang technisch durchgeführt, insbesondere bei der Herstellung
von Azofarbstoffen. Bei in Wasser schwerlöslichen Aminen treten
dabei Probleme auf infolge der geringen Löslichkeit der Amine. In der
Praxis wird deshalb das Amin häufig durch Mahlen in eine feinverteilte Form überführt. Aber auch die so hergestellten Suspensionen des
Amins reagieren noch ziemlich langsam, vor allem bei den üblichen
Temperaturen der Diazotierung von 0 - 30°C, insbesondere 0 - 5°C.
Die gebildeten Diazoniumsalze sind mehr oder weniger leicht zersetzlich. Da die Zersetzungsprodukte die Qualität der Azofarbstoffe nachteilig beeinflussen wirken sich lange Reaktionszeiten ungünstig auf
die Farbstoffe aus.

Der vorliegenden Erfindung lag die Aufgabe zugrunde, die aufgeführten
Nachteile der bekannten Verfahren zur Diazotierung von in Wasser
schwerlöslichen Aminen zu beseitigen oder zumindest zu verringern.

Diese Aufgabe wird durch das erfindungsgemässe Verfahren gelöst,
indem man aus einer Schmelze oder Lösung des Amins dieses durch Vermischen mit Wasser ausfällt und dann diazotiert. Bei diesem Vorgehen
kann man überraschenderweise auf das aufwendige Mahlen verzichten
und dennoch das Diazotierungsmittel erheblich schneller zugeben, ohne
dass Nitritzersetzung auftritt. Die Reaktionszeit für die Diazotierung ist deutlich kürzer und die Diazoverbindungen werden in gleicher,
oft sogar in höherer Reinheit als nach dem üblichen Verfahren erhalten.

Die vorliegende Erfindung betrifft somit ein Verfahren zur Diazotierung von in Wasser schwerlöslichen primären aromatischen Aminen, welches dadurch gekennzeichnet ist, dass man zunächst eine Schmelze oder Lösung des Amins herstellt, aus dieser das Amin anschliessend durch Vermischen mit Wasser ausfällt und bei einer Temperatur von 10 bis 100°C während 0,1 bis 20 Minuten das Amin durch Umsetzung mit einem handelsüblichen Diazotierungsmittel diazotiert.

Die Diazotierung wird vorzugsweise adiabatisch durchgeführt. Die bevorzugte Temperatur liegt bei 20 - 70°C, insbesondere 20 - 50°C.

Unter adiabatischer Umsetzung wird hier eine Umsetzung verstanden, bei der die Reaktionsmischung weder gekühlt noch geheizt wird und auch keine Reaktionsgefässe verwendet werden, die besonders gegen Wärmeübertragung isoliert sind.

Die Dauer der Diazotierung hängt u.a. von der Art des Amins und der Temperatur ab, vorzugsweise beträgt sie 0,1 bis 10 Minuten.

Als Lösungsmittel für das Amin kommen wasserlösliche Säuren oder wasserlösliche organische Lösungsmittel in Betracht.

Geeignete Säuren sind z.B. Schwefelsäure, Phosphorsäure oder Essigsäure und als organische Lösungsmittel kommen z.B. Verbindungen mit Hydroxyl-, Carbonyl-, Carboxyl-, Sulfon- oder Sulfosäuregruppen oder polare aprotische Verbindungen in Betracht. Geeignete Lösungsmittel sind z.B. Methanol, Ethanol, Aceton, Dimethylsulfoxid, Sulfolan, Sulfolen, Dimethylformamid, Tetramethylharnstoff, N-Methylpyrrolidon oder Acetonitril. Es können auch Gemische dieser organischen Lösungsmittel oder Gemische aus den Säuren und organischen Lösungsmitteln eingesetzt werden.

Vorzugsweise verwendet man Schwefelsäure, Eisessig, Dimethylsulfoxid, Dimethylformamid, Sulfolan, Tetramethylharnstoff oder N-Methylpyrrolidon.

Es ist zweckmässig, das Amin bei erhöhter Temperatur in der Säure oder dem organischen Lösungsmittel zu lösen, vorzugsweise bei 30 bis 150°C, insbesondere bei 50 bis 100°C.

- 3 -

Falls das Amin als Schmelze eingesetzt wird, so weist diese vorzugsweise ebenfalls eine Temperatur von 30 bis 150°C, insbesondere 50 bis 100°C auf.

Als Amine sind für das erfindungsgemässe Verfahren in Wasser schwer lösliche aromatische Amine geeignet. Es handelt sich dabei um die aus der Chemie der Azofarbstoffe bekannten, von Sulfogruppen freien Diazokomponenten, vorzugsweise um Derivate des Anilins, Naphthylamins oder von heterocyclischen aromatischen Aminen. In Frage kommen z.B.:

2-Nitroanilin, 4-Nitroanilin, 2-Chlor-4,6-dinitroanilin, 4-Nitro-1-naphthylamin, 4-Chlor-1-naphthylamin, 2,5-Dichloranilin, 3,3'-Dichlorbenzidin, 5-Nitro-2-aminoanisol, 3-Nitro-4-aminotoluol, 4-Chlor-2-nitro-anilin, 4-Chloranilin, 2,4-Dichloranilin, 3-Nitro-4-aminoanisol, 2-Chlor-4-nitroanilin, 2-Amino-anisol-4-sulfodiethylamid, 5-Chlor-2-amino-toluol, 4-Chlor-2-amino-toluol, 4-Nitro-2-amino-toluol, 5-Nitro-2-amino-toluol, 4-Nitro-2-amino-anisol, 3,3'-Dimethoxybenzidin, 3,3'-Dimethoxy-6,6'-dichlorbenzidin, 2-Amino-4-chlorphenol, 2-Aminophenol-4-sulfamid, 2-Aminophenol-5-sulfamid, 2-Aminophenol-4-sulfomethylamid, 3-Amino-4-hydroxyphenylmethylsulfon, 2-Amino-5-nitrophenylmethyl-sulfon, 4-Amino-3-nitrophenylmethylsulfon, 2-(N-Methyl-N-cyclo-hexylsulfonamido)-anilin, 3-Amino-1,2,4-triazol, 2-Aminothiazol, 2-Amino-4,2',4'-trichlordiphenylether, 2-Amino-6-methoxy-1,3-benzo-thiazol und 4-Amino-azobenzol.

Das erfindungsgemässe Verfahren eignet sich vorzugsweise zur Diazotierung von Anilinen, welche ein- oder mehrmals durch Nitro, Chlor oder N,N-Dialkylsulfonamid substituiert sind, wobei unter Alkyl geradkettiges, verzweigtes oder cyclisches $C_1$-$C_6$-Alkyl zu verstehen ist.

Für das erfindungsgemässe Verfahren besonders gut geeignete Amine sind 2-Chlor-4-nitroanilin, 4-Chlor-2-nitroanilin und 2-(N-Methyl-N-cyclohexylsulfonamido)-anilin.

Die Schmelze oder Lösung des Amins wird mit Wasser zusammengebracht, vorzugsweise mit Eis oder einer Eis/Wasser-Mischung, wobei
das Amin praktisch vollständig ausfällt. Man kann dabei Wasser zu der
Schmelze oder Lösung des Amins geben, vorzugsweise lässt man jedoch die
Schmelze oder Lösung des Amins in Wasser bzw. Eis/Wasser-Mischung einlaufen. Das Wasser kann dabei weitere Zusätze enthalten, z.B. geeignete
oberflächenaktive Hilfsmittel und/oder die für die Diazotierung benötigte
Säure. Vorzugsweise enthält das Wasser ein Gemisch aus einem handelsüblichen anionischen und einem nichtionischen Hilfsmittel sowie so viel
Säure, wie insgesamt für die Diazotierung erforderlich ist. Falls das Amin
in Säure gelöst wurde, so ist diese Säuremenge selbstverständlich zu berücksichtigen.

Als oberflächenaktive Hilfsmittel kommen die üblichen anionischen und/oder
nichtionischen in Betracht. Als nichtionische Hilfsmittel werden vor
allem Kondensationsprodukte aus Alkylphenolen und Ethylenoxid und als
anionische Hilfsmittel vor allem Kondensationsprodukte aus aromatischen
Sulfonsäuren und Formaldehyd verwendet. Bevorzugt sind Kondensationsprodukte aus $C_5$-$C_{15}$-Alkylphenolen mit 5 - 15 Mol Ethylenoxid, vorzugsweise
$C_8$-$C_{12}$-Alkylphenolen mit 8 - 12 Mol Ethylenoxid sowie Kondensationsprodukte aus Naphthalinsulfonsäuren mit Formaldehyd, vorzugsweise
Naphthalinmonosulfonsäuren mit Formaldehyd.

Als Diazotierungsmittel können alle Agentien, die im Reaktionsgemisch salpetrige Säure entstehen lassen, z.B. Salpetrigsäureester
oder Nitrosylverbindungen, wie Nitrosylschwefelsäure oder Nitrosylhalogenide verwendet werden. Vorzugsweise werden Nitrite, insbesondere Natriumnitrit, in Verbindung mit Säure, vor allem Mineralsäure, eingesetzt.

Das erfindungsgemässe Verfahren kann diskontinuierlich oder vorzugsweise kontinuierlich durchgeführt werden. In diesem Falle stellt
man zunächst eine Schmelze oder Lösung des Amins her, fällt dieses durch Vermischen mit Wasser wieder aus und bringt die erhaltene
Suspension kontinuierlich mit dem Diazotierungsmittel zur Reaktion,
wobei die erforderliche Säure zusammen mit der Aminsuspension
oder über eine getrennte Leitung zugeführt wird. Es ist auch möglich,
die Schmelze oder Lösung des Amins und die gegebenenfalls benötigte
Säure direkt mit der Lösung des Nitrits zusammenzubringen, so
dass das Amin evt. zum Teil diazotiert wird, ohne zunächst wieder auszufallen.

Vorzugsweise leitet man die Aminsuspension und die Nitritlösung zunächst in eine Mischkammer oder einen kleinen Rührkessel, wo die
Edukte intensiv miteinander vermischt werden, wobei bereits zum
Teil eine Diazotierung stattfindet. Anschliessend wird die Reaktionsmischung in einen Reaktor, vorzugsweise einen Rohrreaktor, geleitet, und dort die Umsetzung zu Ende geführt.

Nach dem erfindungsgemässen Verfahren erhält man eine Lösung der
Diazokomponenten, welche nur sehr wenig oder gar keine unlöslichen
Rückstände (Amin) enthält.

Eine bevorzugte Ausführungsform des erfindungsgemässen Verfahrens
besteht darin, dass man die erhaltene Lösung der Diazokomponenten
kontinuierlich mit einer geeigneten Kupplungskomponente zur Reaktion bringt, z.B. in einem geeigneten Rohrreaktor, wobei gleichzeitig eventuell benötigte weitere Zusätze, z.B. Basen zur Erzielung eines weniger sauren pH-Wertes, hinzugefügt werden können.

Die Bedingungen bei der Kupplung entsprechen den üblichen Bedingungen und als Kupplungskomponente kommen die bekannten Verbindungen
in Betracht, vorzugsweise Phenole, Naphthole, Aniline, Naphthylamine, Pyrazolone oder Acetessigsäureamide.

Die Azofarbstoffe, welche aus den erfindungsgemäss erhaltenen Diazokomponenten hergestellt werden, zeichnen sich zum Teil durch
höhere Reinheit aus verglichen mit den auf übliche Weise hergestellten gleichen Azofarbstoffen. Dies führt in einigen Fällen zur Verbesserung der Echtheiten der erhaltenen Färbungen.

Die nachfolgenden Beispiele dienen der Erläuterung der Erfindung,
ohne sie darauf zu beschränken. Die Temperaturen sind in Grad
Celsius angegeben.

Beispiel 1:  17,3 g 2-Chlor-4-nitroanilin werden in 20 ml Eisessig
bei 90° gelöst und unter gutem Rühren in einen intensiv durchmischten Kleinreaktor zu einer Mischung aus 100 g Eis, 0,4 g
eines Gemisches aus anionischem und nichtionogenem Hilfsmittel
(Kondensationsprodukt aus Octylphenol und 8 Ethylenoxid sowie
Kondensationsprodukt aus Naphthalinsulfonsäure und Formaldehyd)
sowie 30 ml konzentrierter HCl gegeben. In der erhaltenen Reaktionsmischung ist das Amin praktisch vollständig ausgefällt. Zu der
Reaktionsmischung gibt man bei 20° im Verlauf von 18 Sekunden
25 ml 4 N Natriumnitritlösung und rührt noch 1 Minute nach. Nach dieser Zeit ist die Diazotierung praktisch vollständig beendet. Der
unlösliche Rückstand in der Reaktionslösung beträgt weniger als 1 %.

Beispiel 2:  Vergleichsbeispiel
17,3 g 2-Chlor-4-nitroanilin werden in einem intensiv durchmischten
Kleinreaktor mit 100 g Eis, 0,4 g des vorstehend aufgeführten
Hilfsmittelgemisches, 30 ml konzentrierter HCl und 20 ml Eisessig
während 2 Minuten angeschlämmt. Bei 20° gibt man 25 ml 4 N Natriumnitritlösung während 2 Minuten zu. Schnellere Zugabe der Nitritlösung ist zu vermeiden, da sie deutliche Zersetzung des Nitrits
zur Folge hat. Man rührt noch 3,5 Minuten nach und erhält dann eine
Reaktionslösung von gleicher Beschaffenheit wie im vorstehenden
Beispiel 1.

Beispiel 3: 17,3 g 2-Chlor-4-nitroanilin werden in 10 ml Dimethylsulfoxid bei 45° gelöst und unter gutem Rühren in einen intensiv
durchmischten Kleinreaktor zu einer Mischung aus 100 g Eis, 0,4 g
des Gemisches aus anionischem und nichtionogenem Hilfsmittel gemäss Beispiel 1 sowie 30 ml konzentrierter HCl gegeben. In der erhaltenen Reaktionsmischung ist das Amin praktisch vollständig ausgefällt. Zu der
Reaktionsmischung gibt man bei 20° im Verlauf von 18 Sekunden 25 ml
4 N Natriumnitritlösung und rührt noch 1 Minute nach. Nach dieser
Zeit ist die Diazotierung praktisch vollständig beendet. Der unlösliche Rückstand in der Reaktionslösung beträgt weniger als 1 %.

Beispiel 4: 17,3 g 4-Chlor-2-nitroanilin werden in 20 ml 96 %iger
Schwefelsäure bei 90 ° gelöst und unter gutem Rühren in einen intensiv durchmischten Kleinreaktor zu einer Mischung aus 100 g Eis
und 0,4 g des Gemisches aus anionischem und nichtionogenem Hilfsmittel gemäss Beispiel 1 gegeben. In der erhaltenen Reaktionsmischung ist das
Amin praktisch vollständig ausgefällt. Zu der Reaktionsmischung
gibt man bei 20° im Verlauf von 18 Sekunden 25 ml 4 N Natriumnitritlösung und rührt noch 1 Minute nach. Nach dieser Zeit ist
die Diazotierung praktisch vollständig beendet. Der unlösliche
Rückstand in der Reaktionslösung beträgt weniger als 1 %.

Beispiel 5: Vergleichsbeispiel

17,3 g 4-Chlor-2-nitroanilin werden in einem intensiv durchmischten Kleinreaktor mit 100 g Eis, 0,4 g des im Beispiel 1 aufgeführten
Hilfsmittelgemisches und 20 ml 96 %iger Schwefelsäure während
2 Minuten angeschlämmt. Bei 20° gibt man 25 ml 4N Natriumnitritlösung während 7 Minuten zu. Schnellere Zugabe der Nitritlösung ist
zu vermeiden, da sie deutliche Zersetzung des Nitrits zur Folge
hat. Man rührt noch 8 Minuten nach und erhält dann eine Reaktionslösung von gleicher Beschaffenheit wie im vorstehenden Beispiel 4.

Beispiel 6: 26,8 g 2-(N-Methyl-N-cyclohexylsulfonamido)-anilin werden in 20 ml Eisessig bei 80° gelöst und unter gutem Rühren in
einen intensiv durchmischten Kleinreaktor zu einer Mischung aus 100 g Eis,
0,4 g des Gemisches aus anionischem und nichtionogenem Hilfsmittel gemäss
Beispiel 1 sowie 30 ml konzentrierter HCl gegeben. In der erhaltenen Reaktionsmischung ist das Amin praktisch vollständig ausgefällt. Zu der Reaktionsmischung gibt man bei 20° im Verlauf von 1,5
Minuten 25 ml 4 N Natriumnitritlösung und rührt noch 2 Minuten nach.
Nach dieser Zeit ist die Diazotierung praktisch vollständig beendet. Der unlösliche Rückstand in der Reaktionslösung beträgt
weniger als 1 %.

Beispiel 7: Vergleichsbeispiel
26,8 g 2-(N-Methyl-N-cyclohexylsulfonylamido)-anilin werden in
einem intensiv durchmischten Kleinreaktor mit 100 g Eis, 0,4 g des im
Beispiel 1 aufgeführten Dispergatorgemisches, 30 ml konzentrierter
HCl und 20 ml Eisessig während 2 Minuten angeschlämmt. Bei 20°
gibt man 25 ml 4 N Natriumnitritlösung während 6 Minuten zu.
Schnellere Zugabe der Nitritlösung ist zu vermeiden, da sie deutliche Zersetzung des Nitrits zur Folge hat. Man rührt noch
5 Minuten nach und erhält dann eine Reaktionslösung von gleicher
Beschaffenheit wie im vorstehenden Beispiel 6.

Beispiel 8: 173 g 2-Chlor-4-nitroanilin werden in 200 ml Eisessig
bei 85° gelöst und dann unter gutem Rühren in einem intensiv gerührten Kleinreaktor auf eine Mischung aus 1000 ml Eiswasser, 300 ml
konzentrierter Salzsäure, 2 g des nichtionogenen und 2 g des anionischen
Hilfsmittels gemäss Beispiel 1 gegeben. Das Amin fällt dabei in
Form einer reaktiven Suspension an. Diese Suspension wird durch
Zugabe von Wasser auf ein Volumen von 2000 ml aufgefüllt. Die
Temperatur beträgt ca. 20°.

Von dieser Suspension werden kontinuierlich 200 ml/min. in einen Rührkessel von ca. 100 ml Volumen geleitet, in den gleichzeitig über eine getrennte Zuleitung kontinuierlich 25 ml/min. 4 N Natriumnitritlösung eingespeist werden. Die Reaktionsmischung fliesst aus dem Rührkessel durch einen Rohrreaktor von 12 m Länge und 6 mm Durchmesser und verlässt diesen mit einer Temperatur von ca. 27° als klare Lösung, was bedeutet, dass das Amin vollständig diazotiert wurde.

Beispiel 9: In einem ersten Vorratsgefäss werden 865 g 4-Chlor-2-nitroanilin in 1000 ml 98 %iger Schwefelsäure bei 85° gelöst.

In einem zweiten Vorratsgefäss werden 1000 ml 4 N Natriumnitritlösung mit 5000 ml Eiswasser gemischt. Aus den beiden Vorratsgefässen werden gleichzeitig, über getrennte Leitungen kontinuierlich 55,6 g/min. der Aminlösung und 150 ml/min. der Nitritlösung in einen kleinen Rührkessel von ca. 100 ml Volumen geleitet. Die Reaktionsmischung fliesst aus dem Rührkessel durch einen Rohrreaktor von 12 m Länge und 6 mm Durchmesser und verlässt diesen mit einer Temperatur von ca. 30° als klare Lösung, was bedeutet, dass das Amin vollständig diazotiert wurde. Die Diazolösung wird in einem Zwischenbehälter aufgefangen und dort überschüssiges Nitrit mit Sulfaminsäure zerstört.

In einem seperaten Behälter wird die Lösung der Kupplungskomponente zubereitet, indem man 910 g 1-Ethyl-3-cyano-4-methyl-6-hydroxy-pyridon-2, 60 g Natriumacetat, in ca. 4000 ml Wasser unter Zugabe von NaOH conc. zur Einstellung eines pH-Wertes von 7,5 löst und anschliessend die Lösung bis zu einem Volumen von 5000 ml mit Wasser auffüllt.

Ueber getrennte Zuleitungen speist man bei 30° in einen kontinuierlich arbeitenden Kleinreaktor 197 ml/min. der Diazolösung und 100 ml/min.

der Lösung der Kupplungskomponente.

Bei einer Verweilzeit von $<$1 Minute und 30 - 40° erhält man den Azofarbstoff mit einer Ausbeute von über 98 %.

Patentansprüche

1. Verfahren zur Diazotierung von in Wasser schwerlöslichen primären aromatischen Aminen, dadurch gekennzeichnet, dass man zunächst eine Schmelze oder Lösung des Amins herstellt, aus dieser das Amin anschliessend durch Vermischen mit Wasser ausfällt und bei einer Temperatur von 10 bis 100°C während 0,1 bis 20 Minuten das Amin durch Umsetzung mit einem handelsüblichen Diazotierungsmittel diazotiert.

2. Verfahren gemäss Anspruch 1, dadurch gekennzeichnet, dass man die Diazotierung adiabatisch bei 20 - 70°C, insbesondere 20 - 50°C durchführt.

3. Verfahren gemäss einem der Ansprüche 1 und 2, dadurch gekennzeichnet, dass man die Diazotierung während 0,1 bis 10 Minuten durchführt.

4. Verfahren gemäss einem der Ansprüche 1 - 3, dadurch gekennzeichnet, dass man eine Lösung des Amins in einer wasserlöslichen Säure oder einem wasserlöslichen organischen Lösungsmittel einsetzt.

5. Verfahren gemäss Anspruch 4, dadurch gekennzeichnet, dass man als wasserlösliche Säure Schwefelsäure oder Eisessig verwendet.

6. Verfahren gemäss Anspruch 4, dadurch gekennzeichnet, dass man als wasserlösliches Lösungsmittel Dimethylsulfoxid, Dimethylformamid, Sulfolan, Tetramethylharnstoff oder Methylpyrrolidon verwendet.

7. Verfahren gemäss einem der Ansprüche 1 - 6, dadurch gekennzeichnet, dass man eine Schmelze oder Lösung des Amins von 30 bis 150°C, vorzugsweise 50 bis 100°C einsetzt.

8. Verfahren gemäss einem der Ansprüche 1 - 7, dadurch gekennzeichnet, dass man als Amin ein Derivat des Anilins, Naphthylamins oder eines heterocyclischen Amins einsetzt.

9. Verfahren gemäss Anspruch 8, dadurch gekennzeichnet, dass man ein Anilin einsetzt, welches ein- oder mehrmals durch Nitro, Chlor oder N,N-Dialkylsulfonamid substituiert ist.

10. Verfahren gemäss Anspruch 9, dadurch gekennzeichnet, dass man 2-Chlor-4-nitroanilin, 4-Chlor-2-nitroanilin oder 2-(N-Methyl-N-cyclohexylsulfonamido)-anilin einsetzt.

11. Verfahren gemäss einem der Ansprüche 1 - 10, dadurch gekennzeichnet, dass man die Schmelze oder Lösung des Amins in Wasser oder eine Eis/Wasser-Mischung gibt.

12. Verfahren gemäss Anspruch 11, dadurch gekennzeichnet, dass das Wasser bzw. die Eis/Wasser-Mischung ein oberflächenaktives Hilfsmittel enthält.

13. Verfahren gemäss Anspruch 12, dadurch gekennzeichnet, dass man eine Mischung aus einem anionischen und einem nichtionogenen Hilfsmittel verwendet.

14. Verfahren gemäss Anspruch 13, dadurch gekennzeichnet, dass man ein Kondensationsprodukt aus einem $C_8$-$C_{12}$-Alkylphenol mit 8 - 12 Mol Ethylenoxid sowie ein Kondensationsprodukt aus einer Naphthalinmonosulfosäure und Formaldehyd verwendet.

15. Verfahren gemäss einem der Ansprüche 1 - 14, dadurch gekennzeichnet, dass man als Diazotierungsmittel ein Nitrit, vorzugsweise Natriumnitrit, zusammen mit einer Säure verwendet.

16. Verfahren gemäss einem der Ansprüche 1 - 15, dadurch gekennzeichnet, dass man eine Schmelze oder Lösung des Amins herstellt, dieses durch Vermischen mit Wasser wieder ausfällt und die erhaltene Suspension kontinuierlich mit einem Diazotierungsmittel zur Reaktion bringt.

17. Verfahren gemäss Anspruch 16, dadurch gekennzeichnet, dass man kontinuierlich die Aminsuspension in einer Mischkammer mit dem Diazotierungsmittel vermischt und die Reaktionsmischung anschliessend in einen Reaktor, vorzugsweise einen Rohrreaktor, leitet und dort die Reaktion zu Ende führt.

18. Verfahren gemäss Anspruch 17, dadurch gekennzeichnet, dass man die erhaltene Lösung der Diazokomponenten anschliessend kontinuierlich mit einer Kupplungskomponenten zu einem Azofarbstoff umsetzt.